# EUROPÄISCHE PATENTSCHRIFT

(11) **EP 2 289 585 B1**
(45) Veröffentlichungstag und Bekanntmachung des Hinweises auf die Patenterteilung: **21.03.2012**
(21) Anmeldenummer: 10172828.5
(22) Anmeldetag: 13.08.2010
(51) Int. Cl.: A61M 16/04, A61M 25/02, F16L 33/00

(54) **Kanülenschild mit verbesserter Arretierung**
Cannula shield with improved arresting
Collerette de canule dotée d'un arrêt amélioré

(30) Priorität: 31.08.2009 DE 102009029048
(43) Veröffentlichungstag der Anmeldung: 02.03.2011
(73) Patentinhaber: Tracoe Medical GmbH, 60528 Frankfurt/Main (DE)
(72) Erfinder: Schnell, Ralf, 63500, Seligenstadt (DE)
(74) Vertreter: WSL Patentanwälte Partnerschaftsgesellschaft

(56) Entgegenhaltungen:
- EP-A1- 1 407 796
- WO-A1-2008/003929
- DE-A1- 3 223 592
- DE-A1- 19 739 103
- GB-A- 2 227 941
- US-A- 4 774 944
- US-A- 5 806 516
- US-A- 6 010 484
- US-A1- 2008 087 281
- US-B1- 6 634 359

## Beschreibung

Die vorliegende Erfindung betrifft eine Trachealkanüle, welche ein Kanülenrohr mit einem an dem Kanülenrohr angebrachten Schild aufweist, wobei der Schild mit Hilfe einer mindestens für eine begrenzte axiale Verschiebung lösbaren Befestigungseinrichtung in Längsrichtung des Kanülenrohrs in verschiedenen Positionen fixierbar an der Kanüle angebracht ist und im Gebrauch durch die Anlage des Schilds am Hals des Patienten die Position des Kanülenrohrs bzw. des Endes des Kanülenrohrs in der Luftröhre definiert wird.

Tracheotomiekanülen werden eingesetzt, um Patienten, deren obere Atemwege aufgrund einer Krankheit oder eines Unfalls temporär oder dauerhaft blockiert sind, ein (selbstständiges) Atmen zu ermöglichen. Dafür wird unterhalb des Kehlkopfes eine künstliche Öffnung der Luftröhre nach außen erzeugt, die Tracheostoma genannt wird. Die Tracheotomiekanüle wird durch die künstliche Öffnung im Hals in die Luftröhre eingeschoben. In eingebrachtem Zustand verläuft ein unterer Abschnitt der Tracheotomiekanüle im Wesentlichen senkrecht entlang der Luftröhre und dann in einer sanften Biegung (Biegeradius zwischen 1 und 10 cm, vorzugsweise zwischen 2 und 6 cm) um ca. 90°bis 120° durch das Tracheostoma des Patienten nach außen.

Es werden Tracheotomiekanülen verwendet, die der Anatomie des Patienten angepasst sind d. h., es werden auf dem Markt Tracheotomiekanülen mit unterschiedlichen Außendurchmessern des Kanülenrohrs, unterschiedlichen Längen des Kanülenrohrs etc. angeboten.

Teilweise können jedoch die anatomischen Gegebenheiten bei den jeweiligen Patienten sehr verschieden sein, so dass beispielsweise der Abstand der Luftröhre von der Hautoberfläche des Halses bei in etwa gleichem Luftröhrendurchmesser von Patient zu Patient stark variieren kann.

Aus diesem Grund sind Tracheotomiekanülen bekannt, bei denen ein außen an dem Halsschnitt anliegender Schild relativ zur Längsrichtung des Kanülenrohrs verschiebbar und fixierbar angebracht ist. Dadurch lässt sich im oberen Bereich der Tracheotomiekanüle, der teilweise nach außen ragt, die Länge des Abschnitts variieren, der zwischen Halsschnitt und Luftröhrenschnitt bzw. der Biegung der Tracheotomiekanüle in der Luftröhre liegt

Es sind dafür unterschiedliche Möglichkeiten der Arretierung bzw. der Fixierung eines Schildes an dem Kanülenrohr bekannt Es besteht dabei jedoch grundsätzüch die Gefahr, dass die möglicherweise als störend empfundene Tracheotomiekanüle beispielsweise im Schlaf unbeabsichtigt manipuliert wird. Dadurch kann es zu einem ungewollten Öffnen des Fixierungsmechanismus kommen, wodurch sich das Kanülenrohr relativ zum Schild verschieben kann. Ein solches Verschieben ist unkontrolliert und kann zu Versetzungen der Luftröhre führen und im schlimmsten Fall zu einer eingeschränkten Beatmungsfunktion der Tracheotomiekanüle. Eine weitere Gefahr besteht in einem ungewollten Entfernen des Kanülenrohrs aus dem Tracheostoma, was mit Erstickungsgefahr verbunden ist.

Bei Verwendung eines schwergängigen Mechanismusses der Fixierung besteht ein Problem eines fixierbar angebrachten Kanülenschilds an einem Kanülenrohr darin, dass ein solcher Mechanismus zwar ein unbeabsichtigtes Öffnen verhindern kann, möglicherweise aber bei der Betätigung zu ruckartigen Bewegungen führt, wodurch dem Patienten womöglich Schmerzen zugefügt werden. Dabei kann es sogar zu einer Schädigung der Luftröhrenöfnung bzw. der Luftröhre kommen.

Die WO 2008/003929 offenbart einen Schild für eine Tracheostomiekanüle mit einem Verschlussmechanismus mit zwei C-förmigen Klammemrarmen, die an einem Ende schwenkbar zusammenhängen.

Die DE 19 739 103 A1 zeigt einen Schild für eine Tracheostomiekanüle, der in Längsrichtung der Kanüle in verschiedenen Positionen fixierbar an der Kanüle angebracht ist

Ausgehend von diesem Stand der Technik liegt der vorliegenden Erfindung die Aufgabe zugrunde, eine Tracheotomiekanüle mit einem verschiebbaren und fixierbaren Schild zur Verfügung zu stellen, wobei ein unbeabsichtigtes Lösen der Befestigungseinrichtung verhindert werden soll.

Diese Aufgabe wird erfindungsgemäß gelöst durch eine Tracheotomiekanüle, welche ein Kanülenrohr mit einem an dem Kanülenrohr angebrachten Schild aufweist, wobei der Schild in Längsrichtung des Kanülenrohrs in verschiedenen Positionen fixierbar an der Kanüle angebracht ist und im Gebrauch durch die Anlage des Schilds am Hals des Patienten die Position des Kanülenrohrs bzw. des Endes des Kanülenrohrs in der Luftröhre des Patienten definiert wird, dadurch gekennzeichnet, dass für das Fixieren des Schildes eine Befestigungseinrichtung und eine Arretiereinrichtung vorgesehen sind, wobei die Befestigungseinrichtung mindestens ein Betätigungselement aufweist und mit der Außenseite des Kanülenrohrs in lösbaren Klemmeingriff bringbar ist und die Arretiereinrichtung ebenfalls mindestens ein Betätigungselement aufweist und mit der Befestigungseinrichtung in lösbaren Eingriff bringbar ist und den Klemmeingriff sichert und/oder verstärkt.

Unter Klemmeingriff versteht man dabei, dass mindestens zwei Teile an im Wesentlichen einander gegenüberliegenden Bereichen des Kanülenrohrs mit diesem von außen kraft- bzw. reibschlüssig in Eingriff treten. Die Teile können dabei senkrecht zu der Längsachse des Kanülenrohrs in einer Ebene liegen bzw. gegenüber einer solchen Ebene in unterschiedliche Richtungen leicht verschoben sein. Eine solche Klemmvorrichtung ist nach dem Lösen des Klemmeingriffs stufenlos an dem Kanülenrohr verschiebbar und befestigbar und es werden dabei keine besonderen Anforderungen an ein Kanülenrohr gestellt. Das Kanülenrohr kann im Wesentlichen glatt sein und Profilierungen oder Raststellen oder ähnliche Vorrichtungen sind nicht erforderlich, soweit der Reibschluss zwischen Klemmvorrichtung und Kanülenrohr ausreichend ist.

Unter "Sichern" des Klemmeingriffs versteht man, dass die Arretiereinrichtung so mit der Befestigungseinrichtung lösbar in Eingriff bringbar ist, dass ein Lösen der Befestigungseinrichtung bzw. ein Verschieben der Befestigungseinrichtung in Längsrichtung des Kanülenrohrs durch die Arretiereinrichtung verhindert wird.

Die Aussage "den Klemmeingriff verstärkt" umschreibt, dass durch die Arretiereinrichtung die Kraft, die die Befestigungseinrichtung auf das Kanülenrohr ausübt, um den Klemmeingriff zu erzeugen, erhöht wird. Dadurch wird der Halt der Befestigungseinrichtung und/oder der Arretiereinrichtung auf dem Kanülenrohr verbessert.

Die Betätigungselemente der Befestigungseinrichtung und der Arretiereinrichtung sind Elemente, die zum Befestigen oder Lösen der Befestigungseinrichtung oder der Arretiereinrichtung per Hand oder mit Hilfe eines Werkzeuges betätigt werden. Es sind dabei verschiedene Arten der Betätigung denkbar, z.B. das Eindrücken eines Knopfes, das Verdrehen oder Schwenken eines Hebels oder Knopfes o.ä. um eine Achse oder auch das Herausziehen eines Knopfes.

In der Regel weisen die Arretiereinrichtung und/oder die Befestigungseinrichtung jeweils genau ein Betätigungselement oder jeweils zwei Betätigungselemente auf. Sofern zwei Betätigungselemente an einer der Einrichtungen vorgesehen sind, können diese so gestaltet sein, dass zum Lösen oder Fixieren einer der Einrichtungen die jeweiligen Betätigungselemente gleichzeitig betätig werden müssen. Ein Vorteil besteht darin, dass ein unbeabsichtigtes Lösen bei der gleichzeitigen Betätigung von zwei Betätigungselementen unwahrscheinlicher ist. Andererseits ist die Handhabung eine Tracheotomiekanüle, bei der die Arretiereinrichtung und die Befestigungseinrichtung je genau ein Betätigungselement aufweisen, einfacher.

Vorzugsweise sind dabei die Befestigungseinrichtung und die Arretiereinrichtung so gestaltet, dass deren Betätigung bei in die Luftröhre eingebrachter Tracheotomiekanüle ohne eine Bewegung der Tracheotomiekanüle relativ zum Hals des Patienten erfolgen kann. Das bedeutet, dass beispielsweise eine Betätigung der Befestigungseinrichtung anhand von Elementen erfolgt, die auf der Seite des Schildes angeordnet sind, die nicht am Hals des Patienten anliegt.

Die Befestigungseinrichtung und/oder die Arretiereinrichtung können fest mit dem Schild verbunden sein und z. B. in dieses integriert sein, es besteht jedoch auch die Möglichkeit, dass das Schild an der Befestigungseinrichtung und/oder an der Arretiereinrichtung lösbar befestigt ist.

Das Schild besteht bevorzugt aus einem Kunststoffmaterial. Infrage kommen dafür u.a. Materialien wie PVC, Polyurethan, TPE, Polycarbonat oder Polysulfon. Das Schild weist in der Regel Flügel zur Befestigung des Kanülenbandes auf. Die Flügel bestehen jeweils aus einem länglichen Abschnitt, der parallel zum Umfang des Halses verläuft und dessen Längsachse von einer Öffnung des Schildes radial nach außen verläuft. Die Flügel weisen weiterhin Ösen zum Einführen eines Kanülenbandes auf. Die Flügel können dabei an dem Schild angebracht sein bzw. einstückig mit diesem verbunden sein und aus dem gleichen Material wie der Schild bestehen.

In einer bevorzugten Ausführungsform sind das mindestens eine Betätigungselement der Befestigungseinrichtung und das mindestens eine Betätigungselement der Arretiereinrichtung voneinander unabhängig betätigbar, wobei die Betätigungselemente der Befestigungs- und der Arretiereinrichtung sich voneinander unterscheiden und vorzugsweise unterschiedliche Betätigungsmechanismen aufweisen.

Die Befestigungseinrichtung und die Arretiereinrichtung weisen in dieser Ausführungsform voneinander unabhängige Betätigungselemente auf, um ein gleichzeitiges unabhängiges Betätigen beider Betätigungselemente praktisch auszuschließen. Vorzugsweise erfolgt die Bewegung der Betätigungselemente beim Lösen des Eingriffs der Befestigungseinrichtung mit dem Kanülenrohr bzw. beim Lösen des Eingriffs der Arretiereinrichtung mit der Befestigungseinrichtung in unterschiedlichen Ebenen, so dass der Benutzer, z. B. der Patient, auch ohne Sicht auf das Schild und die Befestigungs- bzw. Arretiereinrichtung feststellen kann, welche der Einrichtungen gelöst bzw. fixiert wird. Besonders praktisch ist es dabei, wenn sich die Betätigungselemente der Befestigungs- bzw. der Arretiereinrichtung zusätzlich oder auch ausschließlich durch ihre Form voneinander unterscheiden.

Beispielsweise kann das Lösen oder Fixieren der Befestigungseinrichtung durch Betätigung eines Druckknopfes erfolgen, während die Arretiereinrichtung durch eine Drehbewegung gelöst oder fixiert wird. Dabei kann die Arretiereinrichtung mit dem Druckknopf der ersten Befestigungseinrichtung in Eingriff treten und dadurch ein Eindrücken oder Herausziehen desselben verhindern. Es ist auch ein Fixieren der Befestigungseinrichtung durch eine Dreh- oder Kippbewegung denkbar, während die Arretiereinrichtung durch ein Eindrücken oder Herausziehen eines Betätigungselements gelöst wird.

Bevorzugt weist die Arretiereinrichtung ein Betätigungselement in Form eines Hebels auf, der um eine Achse schwenkbar ist. Ein Hebel als Betätigungselement kann über verschiedene Gelenke an einem Schild so angebracht werden, dass er leicht zu betätigen ist, aber nicht durch eine unbeabsichtigte Bewegung des Trägers der Trachoetomiekanüle gelöst wird.

Gemäß einer besonders bevorzugten Ausführungsform ist das Betätigungselement der Arretiereinrichtung über einen Totpunkt bewegbar und/oder die Arretiereinrichtung verrastet nach Überwindung des Totpunkts in einer Position, in welcher die Arretiereinrichtung in Eingriff mit der Befestigungseinrichtung und/oder mit mindestens einem Betätigungselement der Befestigungseinrichtung steht. Durch das Überwinden eines Totpunktes bei der Betätigung der Arretiereinrichtung wird zusätzlich ein unbeabsichtigtes Lösen verhindert. Die Verrastung und/oder der Totpunkt sollten jedoch so gestaltet sein, dass sie zwar bei der Bedienung bemerkbar sind, aber in beiden Bewegungsrichtungen keinen übermäßigen Kraftaufwand erfordern, der möglicherweise eine unbeabsichtigte Bewegung der Tracheotomiekanüle relativ zu dem Hals des Patienten hervorrufen könnte. Bei einer solchen Vorrichtung kann auch das Betätigungselement der Arretiereinrichtung mit der Befestigungseinrichtung und/oder mit dem Betätigungselement der Befestigungseinrichtung verrasten.

Gemäß einer Ausführungsform der Erfindung ist die Arretiereinrichtung mit der Befestigungseinrichtung und/oder dem mindestens einen Betätigungselement der Befestigungseinrichtung so in lösbaren Eingriff bringbar, dass die Befestigungseinrichtung und/oder das mindestens eine Betätigungselement der Befestigungseinrichtung dadurch blockierbar ist.

Gemäß einer weiteren Ausführungsform der Erfindung weist die Befestigungseinrichtung als Betätigungselement einen Druckknopf auf.

Beispielsweise kann, wenn die erste Befestigungseinrichtung einen Druckknopf als Betätigungselement aufweist und das Betätigungselement der Arretiereinrichtung als Hebel ausgestaltet ist, der Hebel der Arretiereinrichtung in eine Aussparung des Druckknopfs einrasten und dadurch ein Eindrücken oder Herausziehen des Druckknopfs und damit ein Lösen der ersten Befestigungseinrichtung verhindern.

Bevorzugt ist die Befestigungseinrichtung in Klemmeingriff mit der Außenseite des Kanülenrohrs vorgespannt. Das bedeutet, dass die Befestigungseinrichtung, solange sie nicht aktiv betätigt wird, in Klemmeingriff mit der Außenseite des Kanülenrohrs steht. Eine Verschiebung des Schildes in Längsrichtung des Kanülenrohrs bzw. der Befestigungseinrichtung in Längsrichtung des Kanülenrohrs ist daher nur dann möglich, wenn die Befestigungseinrichtung mit Hilfe des Betätigungselements betätigt wird und gleichzeitig der Schild bzw. die Befestigungseinrichtung auf dem Kanülenrohr verschoben wird. Dabei kann eine Betätigung beispielsweise durch andauerndes Eindrücken eines Druckknopfes als Betätigungselement erfolgen.

In einer Ausführungsform besteht die Befestigungseinrichtung aus mindestens zwei die Kanüle mindestens teilweise ringförmig umgreifenden Halteteilen, welche relativ zueinander senkrecht zu ihren Ringachsen verschiebbar sind. Das bedeutet, dass die beiden Öffnungen, wenn sie in Flucht miteinander gebracht werden, das Kanülenrohr aufnehmen können, und das Schild bzw. die Befestigungseinrichtung auf der Außenfläche des Kanülenrohrs verschiebbar ist. Nach dem relativen Verschieben senkrecht zur Achse des Kanülenrohrs bzw. senkrecht zur Achse der Öffnungen überlappen diese Öffnungen nur noch teilweise, so dass sie das Kanülenrohr in dem Überlappungsbereich festklemmen. Dadurch wird der Schild bzw. die Befestigungseinrichtung auf dem Kanülenrohr fixiert.

Besonders bevorzugt ist eine Ausführungsform der Erfindung, bei welcher die Befestigungseinrichtung im Wesentlichen aus drei Teilen besteht, wobei zwei der Teile fest miteinander verbunden sind und alternativ auch einstückig zusammenhängen können und gemeinsam ein Gehäuse bilden, welches zwei miteinander fluchtende Öffnungen für das Hindurchführen einer Tracheotomiekanüle aufweist. Dabei ist ein drittes, im Wesentlichen ebenes Klemmteil vorgesehen, das in dem Gehäuse aufgenommen ist und ebenfalls eine Öffnung für die Hindurchführung einer Tracheotomiekanüle hat, welche mindestens teilweise mit den beiden miteinander fluchtenden Öffnungen der ersten und zweiten Teile in Überlappung bringbar ist, so dass die Tracheotomiekanüle alle drei Öffnungen durchgreifen kann, wobei der dritte Teil, nämlich das Klemmteil, in dem Gehäuse derart vorgespannt ist, daß seine Öffnung in eine zu den beiden anderen Öffnungen exzentrische Lage verschoben wird, wodurch die Tracheotomiekanüle in dem überlappenden Teil der drei Öffnungen eingeklemmt wird.

Die beiden das Gehäuse bildenden Teile können zum Beispiel jeweils einen Schnapprand aufweisen und zusammengesteckt werden, während das dritte, im Wesentlichen ebene Klemmteil dazwischen bzw. in einem der Gehäuseteile aufgenommen ist. Die beiden Gehäuseteile können auch, zum Beispiel durch ein Biegescharnier, einstückig miteinander verbunden sein. Die Mehrteiligkeit des Gehäuses dient vor allem dem Zweck, das Klemmteil sicher in dem aus den beiden anderen Teilen gebildeten Gehäuse aufzunehmen, ohne dass es sich aus dem Gehäuse herausbewegen kann.

Zur Erzeugung der exzentrischen Vorspannung weist das ebene Teil vorzugsweise einen Federbügel auf, der sich an der Innenwand des von dem ersten und zweiten Teil gebildeten Gehäuses abstützt. Der Federbügel kann zum Beispiel aus einem entlang eines Kreisbogens gebogenen Bügel bestehen, der mit einem mittleren Abschnitt an einem Ende des Klemmteiles befestigt ist bzw. einstückig mit diesem verbunden ist und zwei seitliche und nach vorn wegstehende, elastisch wegbiegbare Schenkel aufweist. Selbstverständlich kann der Federbügel auch ein separates Teil sein und auch als ein andersartiges Federelement ausgebildet sein.

Auf der dem Federbügel gegenüberliegenden Seite weist das Klemmteil vorzugsweise ein Betätigungselement auf, welches sich durch eine in dem Gehäuse freigelassene seitliche Öffnung hindurch nach außen erstreckt, so dass das Betätigungselement von der Gehäuseaußenseite her gegen die von dem Federbügel ausgeübte Kraft eindrückbar ist, wobei die drei Teile relativ zueinander so angeordnet sind, daß sich beim Eindrücken des Betätigungselementes der Überlappungsbereich der drei Öffnungen vergrößert und gegebenenfalls die drei Öffnungen auch vollständig zur Deckung gebracht werden können, so dass in diesem Zustand die Tracheotomiekanüle, deren Außendurchmesser geringfügig geringer ist als der Innendurchmesser der drei Öffnungen, leicht in den drei miteinander fluchtenden Öffnungen verschoben werden kann, bzw. umgekehrt die Klemmvorrichtung auf der Tracheotomiekanüle leicht verschoben werden kann. Sobald jedoch das Betätigungselement losgelassen wird, drückt der Federbügel die mittlere der zwei Öffnungen, die sich an dem in dem Gehäuse befindlichen Klemmteil befindet, in eine exzentrische Lage, so dass hierdurch die Tracheotomiekanüle eingeklemmt wird.

Gemäß einer anderen Ausführungsform der Erfindung besteht die Befestigungseinrichtung aus mindestens zwei die Kanüle mindestens teilweise ringförmig umgreifenden Halteteilen, wobei die Halteteile um eine dritte Achse, welche parallel zu den beiden Ringachsen liegt, jedoch nicht mit einer dieser Ringachsen zusammenfällt, verschwenkbar ist. Bei dem Verschwenken der beiden Teile relativ zueinander um diese dritte Achse ändert sich der Überlappungsquerschnitt der beiden Öffnungen der Halteteile, so dass dadurch der gewünschte Klemmeffekt erzeugt werden kann. Auch hierbei können die Halteteile in der Weise vorgespannt sein, dass sich der Überlappungsbereich bzw. die durch beide Halteteile definierte Öffnung unter Wirkung der Vorspannung verkleinert, um so die Kanüle festzuklemmen, wobei eine Betätigung der Halteteile, beispielsweise durch Erfassen und Zusammendrücken der entsprechenden Betätigungselemente zwischen einem Finger und dem Daumen einer Hand, gegen die Vorspannung und zwecks Vergrößerung der Öffnung und Verschieben des mit den Halteteilen verbundenen Schildes möglich ist.

Bevorzugt ist weiterhin, dass mindestens ein Teil der Befestigungseinrichtung, der mit dem Kanülenrohr in Eingriff steht, gezahnt ausgebildet ist. Unter einer gezahnten Ausbildung ist dabei zu verstehen, dass die auf dem Kanülenrohr aufliegende Fläche im Querschnitt Rillen bzw. Zacken aufweist. Dadurch wird ein festerer Sitz der Befestigungseinrichtung auf der glatten Oberfläche der Außenseite des Kanülenrohrs ermöglicht.

Gemäß einer Ausführungsform ist der Schild der Tracheotomiekanüle wie bereits oben erwähnt an der Befestigungseinrichtung und/oder der Arretiereinrichtung angebracht. Zweckmäßigerweise befindet sich dabei der Schild relativ zu der Befestigungseinrichtung und der Arretiereinrichtung an der dem Hals des Patienten zugewandten Seite. Die Betätigungselemente zum Lösen und/oder Fixieren der Befestigungseinrichtung und der Arretiereinrichtung, um diese zusammen mit dem Schild entlang der Tracheotomiekanüle verschieben zu können, befinden sich dementsprechend auf der dem Hals eines Patienten abgewandten Seite des Schildes.

Bevorzugt weist dabei der Schild eine Öffnung auf, deren Durchmesser größer ist als der Durchmesser der Tracheotomiekanüle, wobei zumindest die Befestigungseinrichtung einen zylindrischen Ansatz aufweist, und wobei die Öffnung des Schildes zu dem zylindrischen Ansatz der Befestigungseinrichtung im Wesentlichen konzentrisch ausgerichtet ist. Der den Schild tragende zylindrische Ansatz weist dabei an seinem Ende vorzugsweise einen radial nach außen ragenden Halteflansch auf. Dieser Halteflansch dient der Befestigung des Schildes.

Weitere Vorteile, Merkmale und Anwendungsmöglichkeiten der Erfindung ergeben sich aus der folgenden Beschreibung einer bevorzugten Ausführungsform und den dazu gehörigen Figuren. Es zeigen:
- Figur 1: eine schematische Darstellung einer Vorrichtung, die eine Befestigungseinrich- tung und eine Arretiereinrichtung umfasst, wobei die Arretiereinrichtung gelöst ist und die Befestigungseinrichtung betätigt wird,
- Figur 2: die Vorrichtung gemäß Figur 1, wobei die Arretiereinrichtung gelöst ist und die Befestigungseinrichtung nicht betätigt wird, und
- Figur 3: die Vorrichtung gemäß Figur 1, wobei die Arretiereinrichtung eingerastet ist und die Befestigungseinrichtung nicht betätigbar ist.

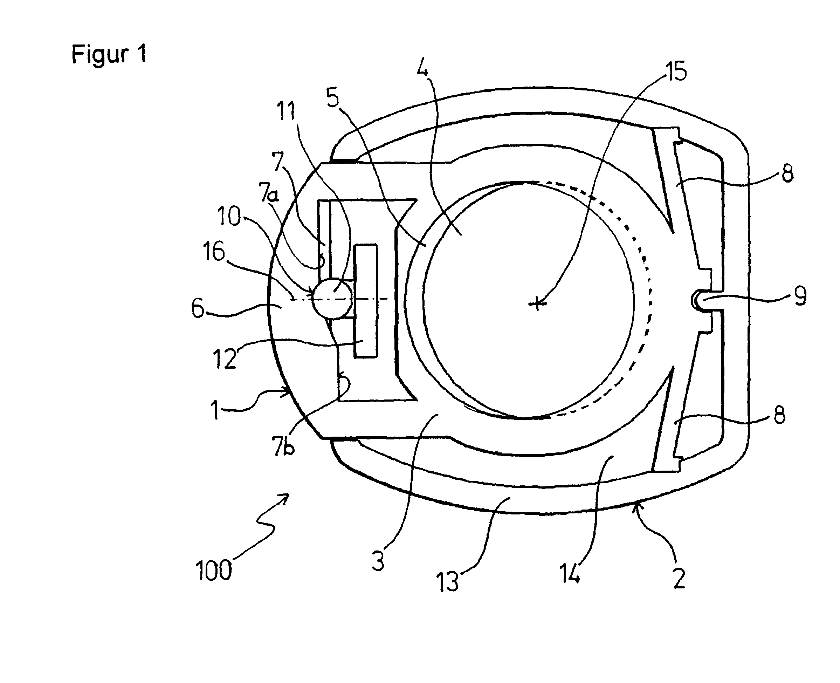

In Figur 1 ist eine geöffnete Schildbefestigung 100 in einer Draufsicht entlang einer gedachten Kanülenachse 15 gezeigt, die eine Befestigungseinrichtung 1 und eine Arretiereinrichtung 10 umfasst. Die Befestigungseinrichtung 1 besteht unter anderem aus einem ein- oder zweiteiligen Gehäuse 2, das eine schmale, schlitzförmige Aussparung 14 definiert, in der ein passend eingesetztes Klemmteil 3 quer zu der Kanülenachse 15 bewegbar ist. In der Darstellung ist eine Grundplatte des Gehäuses mit einem die Aussparung 14 umfassenden Rand 13 und das in der Aussparung 14 liegende Klemmteil 3 gezeigt. Eine Deckplatte als weiterer Gehäuseteil ist in der Darstellung in Figur 1 abgenommen und daher nicht dargestellt.

Die Grundplatte 2 und das Klemmteil 3 weisen jeweils kreisförmige Öffnungen 4 und 5 auf, die so gestaltet sind, dass sie im Wesentlichen miteinander fluchtend angeordnet sind, wenn der Druckknopf 6 eingedrückt wird und das Klemmteil 3 am Anschlag 9 anliegt. Es versteht sich, dass auch die nicht dargestellte Deckplatte eine entsprechende Öffnung aufweist, die genau mit der dargestellten Öffnung 4 der Grundplatte fluchtet. Eine Kanüle, deren Verlauf hier nur durch die Achse 15 wiedergegeben ist, hat den gleichen bzw. einen geringfügig kleineren Durchmesser als die Öffnungen 4, 5, so dass sie sich durch die Öffnungen hindurcherstreckt und in dem annähernd fluchtenden Zustand der Öffnungen leicht in Längsrichtung durch die Öffnungen 4, 5 hindurch bewegbar ist. Der Federbügel 8, der Bestandteil des Klemmteils 3 ist, wirkt einem Druck auf den Druckknopf 6 entgegen. Dieser Federbügel 8 stützt sich an dem Rand 13 der Grundplatte 2 ab. Wird der Druck auf den Druckknopf gelöst, so drücken die Beine des Federbügels 8 das Klemmteil 3 seitlich ein Stück weit aus dem Gehäuse 2 heraus, so dass die zunächst ganz oder fast miteinander fluchtenden Öffnungen 4, 5 gegeneinander verschoben werden und dadurch die sich durch beide Öffnungen 4, 5 erstreckende Kanüle festklemmen. Aus Gründen der besseren Darstellbarkeit der verschiedenen Bauteile ist in Figur 1 der Druckknopf nicht vollständig bis zum Anschlag 9 eingedrückt.

Der nicht dargestellte Gehäuseteil, die Deckplatte, kann einen Flansch oder ein anderes Element aufweisen, das der Befestigung eines Kanülenschilds dient. Alternativ kann auch die Grundplatte des Gehäuses 2 auf der in Figur 1 nicht einsehbaren Seite einen Flansch oder ein anderes Element aufweisen, das der Befestigung des Kanülenschilds dient. Dabei ist der Flansch oder das Element so angeordnet, dass es konzentrisch zu der Öffnung des jeweiligen Gehäuseteiles angebracht ist und das Kanülenschild mit seiner Öffnung ebenfalls konzentrisch zu diesen Öffnungen angebracht werden kann. In dieser Anordnung kann das Kanülenrohr durch die Öffnungen des Kanülenschilds, des Flansches oder anderen Elements der dargestellten Vorrichtung und der Gehäuseteile geführt werden, wenn der Druckknopf vollständig eingedrückt ist.

Bei der in dieser Figur dargestellten Ausführungsform stellt die Befestigungseinrichtung 1 eine Klemmverbindung dar, die durch Druck auf einen Druckknopf 6 als Betätigungselement der Befestigungseinrichtung 1 gelöst werden kann, um nach Bedarf den Schild auf der Kanüle und in deren Längsrichtung zu verschieben.

Die Arretiereinrichtung 10 weist einen verschwenkbaren Hebel 11 auf, der an einem Einschub 12 um eine Achse 16 verschwenkbar gelagert ist, welcher wiederum an der der Grundplatte 2 und der Deckplatte befestigt ist. Der Hebel 11 erstreckt sich in eine asymmetrische Aussparung 7 des Klemmteils 3 hinein und durch diese hindurch. Die asymmetrische Aussparung 7 weist eine einseitige Auflaufschräge 17 auf, auf welcher der Hebel 11 beim Verschwenken um die Achse 16 entlanggleiten kann. In einer Schwenkstellung hat der Hebel 11 in der Aussparung 7 gegenüber deren Rand 7a genügend Spiel, so dass der Druckknopf 6 bis zum Anschlag eingedrückt werden kann. Wird er jedoch über die Auflaufschräge 17 hinweg in eine andere Schwenkstellung bewegt, kommt er mit dem dortigen Rand 7b der Aussparung 7 in festen Eingriff, so dass eine Bewegung des Klemmteils 3 relativ zur Grundplatte 2 durch Eindrücken des Druckknopfes 6 nicht mehr möglich ist. Im Gegenteil werden der Druckknopf 6 und mit ihm das Klemmteil 3 durch das Verschwenken des Hebels 11 unter den Rand 7b tendenziell eher noch weiter seitlich aus dem Gehäuse 2 herausgedrückt, so dass die Öffnungen 4, 5 noch etwas weiter gegeneinander verschoben werden bzw. noch fester mit einer sich durch beide Öffnungen hindurcherstreckenden Kanüle in Klemmeingriff treten.

Die nicht dargestellte Deckplatte oder die dargestellte Grundplatte 2 können in einer Ausführungsform als Schild ausgestaltet sein, so dass eine gesonderte Anbringung des Kanülenschilds nicht erforderlich ist. Vorzugsweise weist das Schild dabei Flügel zur Befestigung eines Kanülenbandes auf.

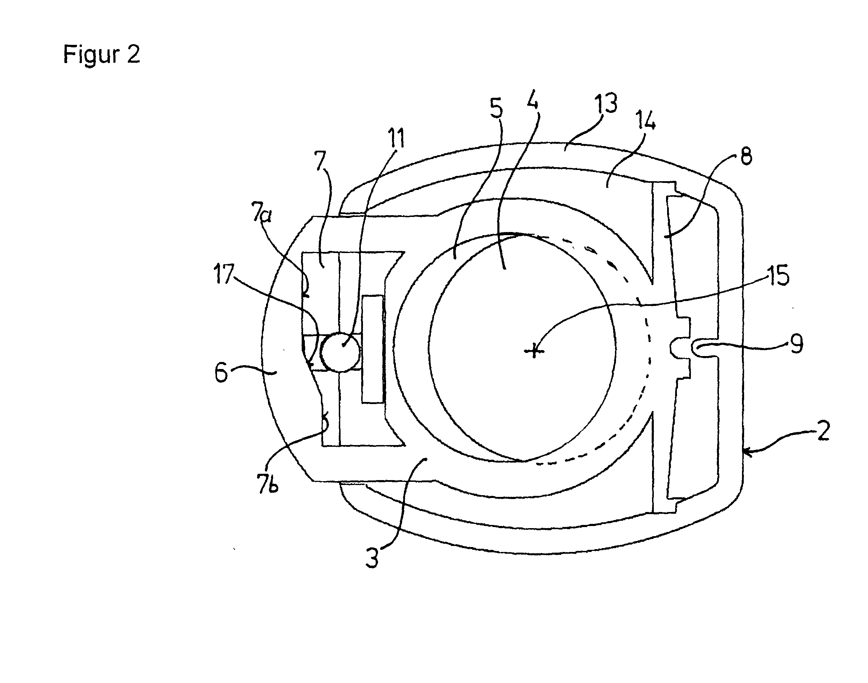

In Figur 2 ist die Vorrichtung aus Figur 1 in einem Zustand gezeigt, in welchem der Druckknopf 6 nicht eingedrückt ist, der Federbügel 8 aber immer noch unter Vorspannung steht. Die Öffnungen 4 und 5 sind nicht mehr miteinander fluchtend angeordnet, und der Querschnitt der verbleibenden freien Öffnung ist etwas kleiner als der Außendurchmesser eines dafür vorgesehenen Kanülenrohrs. Dies bewirkt, dass ein zuvor in die Öffnungen eingeführtes Kanülenrohr in dieser Position durch die Befestigungseinrichtung 1, d. h. das Gehäuse 2 und das Klemmteil 3 bzw. die Ränder der überlappenden Öffnungen 4, 5, fest eingeklemmt wird. Diese Klemmverbindung zwischen Kanüle und Befestigungseinrichtung wird noch sicherer, wenn die Innenränder der Öffnungen 4 und/oder 5 gezackt sind und dadurch die Reibung zwischen den Innenrändern der Öffnungen und der Außenfläche des Kanülenrohres verstärkt wird.

In Figur 2 ist die Arretiereinrichtung 10 so angeordnet, dass sie ein Öffnen der Befestigungseinrichtung 1 nicht verhindert d. h., der Klemmeingriff der Befestigungseinrichtung 1 mit einem hier nicht dargestellten Kanülenrohr ist nicht gesichert. Dazu ist der Hebel 11 in der gleichen Position, d. h. gegenüber dem Rand 7a der Aussparung 7, angeordnet, wie in Figur 1. Daher ist durch unabsichtliches Betätigen des Druckknopfes 6 oder bei größeren Kräften ein Verrutschen der Befestigungseinrichtung 1 auf dem Kanülenrohr nicht gänzlich auszuschließen.

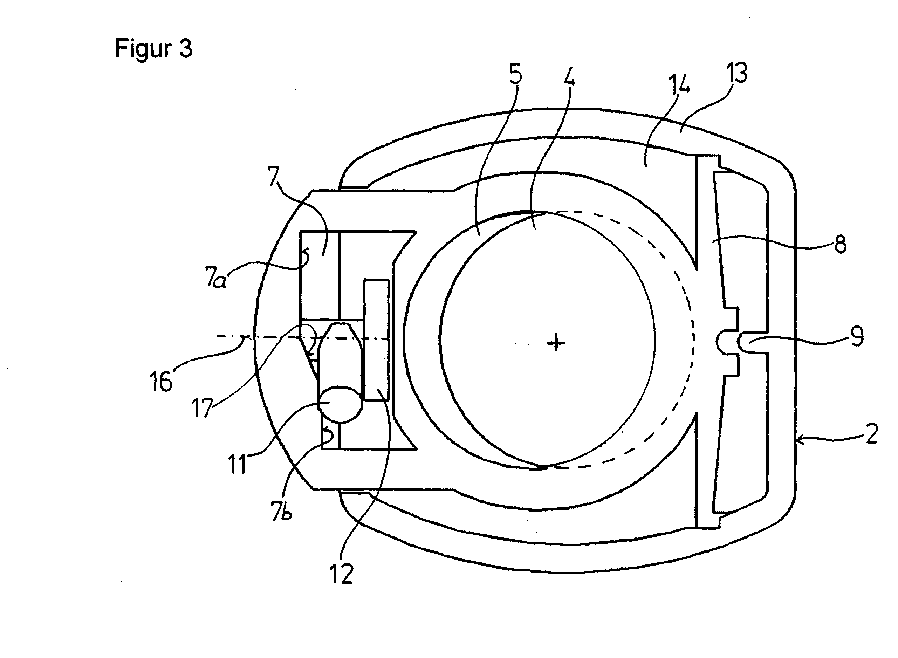

Figur 3 zeigt die Vorrichtung aus den Figuren 1 und 2, wobei der Halt der Befestigungseinrichtung 1 auf der nicht dargestellten Kanüle durch die Fixierung der Arretiereinrichtung 10 noch weiter verstärkt und gesichert ist.

Der Hebel 11 ist dabei auf dem Einschub 12 verschwenkt und in eine zwischen Druckknopf 6 und dem an der Grundplatte 2 befestigten Einschub 12 gebildete asymmetrische Aussparung 7 hineingedrückt worden und liegt an dem Rand 7a der asymmetrischen Aussparung 7 an. Dabei weisen die Grundplatte 2 und die nicht dargestellte Deckplatte die teilweise miteinander fluchtenden Öffnungen auf, wobei die Öffnung 4 der Grundplatte 2 dargestellt ist. Der Druckknopf 6 ist mit dem ebenen Klemmteil 3 verbunden, welches ebenfalls eine Öffnung 5 aufweist. Die Öffnungen 4 und 5 sind bei vollständigem Eindrücken des Druckknopfs 6, wenn das Klemmteil 3 am Anschlag 9 anliegt, ebenfalls miteinander fluchtend angeordnet. Das Kanülenrohr erstreckt sich durch die Öffnungen 4 und 5 und durch die Öffnung der Deckplatte. Die Aussparung 7 ist im Bereich des Randes 7a so gestaltet, dass sie in dem allein durch die Federkraft des Federbügels 8 bereitgestellten Eingriffszustand dem Anschlag 9 näher liegt als die dem Rand 7a zugewandte Oberfläche des Hebels 11, der durch den Einschub 12 in einer festen Position gehalten wird und nur um seine Achse 16 bewegbar ist. Durch Verschwenken des Hebels 11 unter den Rand 7a bzw. zwischen den Rand 7a und die Oberseite des Einschubs 12 werden deshalb die Öffnungen 4 und 5 noch weiter gegeneinander verschoben, so dass der Klemmeingriff am Kanülenrohr verstärkt wird und gleichzeitig ein Eindrücken des Betätigungselements in Form des Druckknopfes 6 verhindert wird.

Für Zwecke der ursprünglichen Offenbarung wird darauf hingewiesen, daß sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen und die Betonung der Unabhängigkeit der einzelnen Merkmale voneinander wird hier nur der Kürze und der Lesbarkeit der Beschreibung wegen verzichtet.

### Liste der Bezugszahlen:

- 1: Befestigungseinrichtung
- 2: Grundplatte (Gehäuseteil)
- 3: Klemmteil
- 4: Öffnung der Grundplatte
- 5: Öffnung des Klemmteils
- 6: Druckknopf
- 7: assymetrische Aussparung in dem Klemmteil
- 7a: Rand der Aussparung 7
- 7b: Rand der Aussparung 7
- 8: Federbügel
- 9: Anschlag
- 10: Arretiereinrichtung
- 11: Hebel
- 12: Einschub
- 13: Rand des Gehäuses
- 14: Aussparung im Gehäuse
- 15: Achse der Kanüle
- 16: Achse des Hebels
- 17: Auflaufschräge

## Patentansprüche

1. Tracheotomiekanüle, welche ein Kanülenrohr mit einem an dem Kanülenrohr angebrachten Schild aufweist, wobei der Schild mit Hilfe einer mindestens für eine begrenzte axiale Verschiebung lösbaren Befestigungseinrichtung in Längsrichtung des Kanülenrohrs in verschiedenen Positionen fixierbar an der Kanüle angebracht ist und im Gebrauch durch die Anlage des Schilds am Hals des Patienten die Position des Kanülenrohrs bzw. des Endes des Kanülenrohrs in der Luftröhre des Patienten definiert wird, wobei die Befestigungseinrichtung mindestens ein Betätigungselement aufweist und mit der Außenseite des Kanülenrohrs in lösbaren Klemmeingriff bringbar ist, **dadurch gekennzeichnet, dass** für das Fixieren des Schildes neben der Befestigungseinrichtung (1) eine Arretiereinrichtung (10) vorgesehen ist und die Arretiereinrichtung ebenfalls mindestens ein Betätigungselement (11) aufweist und mit der Befestigungseinrichtung (1) in lösbaren Eingriff bringbar ist und den Klemmeingriff sichert und/oder verstärkt.

2. Tracheotomiekanüle gemäß Anspruch 1, **dadurch gekennzeichnet, dass** das mindestens eine Betätigungselement der Befestigungseinrichtung (1) und das mindestens eine Betätigungselement (11) der Arretiereinrichtung (10) voneinander unabhängig sind, wobei die Betätigung der Betätigungselemente durch unterschiedliche Mechanismen erfolgt.

3. Tracheotomiekanüle gemäß Anspruch 1 oder Anspruch 2, **dadurch gekennzeichnet, dass** die Arretiereinrichtung (10) als Betätigungselement (11) einen um eine Achse schwenkbaren Hebel aufweist.

4. Tracheotomiekanüle gemäß einem der vorangehenden Ansprüche, **dadurch gekennzeichnet, dass** die Arretiereinrichtung (10) ein Betätigungselement (11) aufweist und das Betätigungselement über einen Totpunkt bewegbar ist und/oder nach Überwindung des Totpunkts in einer Position verrastet, in welcher die Arretiereinrichtung in Eingriff mit der Befestigungseinrichtung (1) und/oder mit mindestens einem Betätigungselement der Befestigungseinrichtung steht.

5. Tracheotomiekanüle gemäß einem der vorangehenden Ansprüche, **dadurch gekennzeichnet, dass** die Arretiereinrichtung (10) mit der Befestigungseinrichtung (1) und/oder mit mindestens einem Betätigungselement der Befestigungseinrichtung in lösbaren Eingriff bringbar ist und die Befestigungseinrichtung und/oder das mindestens eine Betätigungselement der Befestigungseinrichtung **dadurch** blockierbar ist

6. Tracheotomiekanüle gemäß einem der vorangehenden Ansprüche, **dadurch gekennzeichnet, dass** die Befestigungseinrichtung (1) als Betätigungselement einen Druckknopf (6) aufweist

7. Tracheotomiekanüle gemäß einem der vorangehenden Ansprüche, **dadurch gekennzeichnet, dass** die Befestigungseinrichtung (1) in Klemmeingriff mit der Außenseite des Kanülenrohrs vorgespannt ist.

8. Tracheotomiekanüle gemäß einem der vorangehenden Ansprüche, **dadurch gekennzeichnet, dass** die Befestigungseinrichtung (1) aus mindestens zwei die Kanüle mindestens teilweise umgreifenden, ringförmigen Halteteilen besteht, welche relativ zueinander senkrecht zu ihren Ringachsen verschiebbar sind.

9. Tracheotomiekanüle gemäß einem der Ansprüche 1 bis 7, **dadurch gekennzeichnet, dass** die Befestigungseinrichtung (1) aus mindestens zwei die Kanüle mindestens teilweise umgreifenden, ringförmigen Halteteilen besteht, wobei die Halteteile um eine dritte Achse, welche parallel zu den beiden Ringachsen liegt, jedoch nicht mit einer dieser Ringachsen zusammenfällt, verschwenkbar ist.

10. Tracheotomiekanüle gemäß einem der vorangehenden Ansprüche, **dadurch gekennzeichnet, dass** mindestens ein Teil der Befestigungseinrichtung (1), der mit dem Kanülenrohr in Eingriff steht, und/oder ein Teil der Arretiereinrichtung (10) gezahnt ausgebildet ist.

11. Tracheotomiekanüle gemäß einem der vorangehenden Ansprüche, **dadurch gekennzeichnet, dass** der Schild an der Befestigungseinrichtung (1) befestigt ist

12. Tracheotomiekanüle gemäß Anspruch 11, **dadurch gekennzeichnet, dass** der Schild eine Öffnung aufweist, deren Durchmesser größer ist als der Durchmesser der Tracheotomiekanüle und zumindest die Befestigungseinrichtungen (1) einen zylindrischen Ansatz aufweist, wobei die Öffnung des Schilds zu dem zylindrischen Ansatz der Befestigungseinrichtung im Wesentlichen konzentrisch ausgerichtet ist.

13. Tracheotomiekanüle gemäß Anspruch 12, **dadurch gekennzeichnet, dass** das Kanülenrohr aus einer Innenkanüle und einer Außenkanüle besteht, wobei die Innenkanüle durch den Schild und den zylindrischen Ansatz in die Außenkanüle eingeschoben werden kann.

14. Tracheotomiekanüle gemäß einem der Ansprüche 12 bis 13, **dadurch gekennzeichnet, dass** der zylindrische Ansatz an seinem freien Ende einen radial nach außen ragenden Halteflansch aufweist, zur Befestigung des Schilds.

## Claims

1. A tracheotomy cannula which has a cannula tube with a shield mounted to the cannula tube, wherein the shield is mounted fixably on the cannula in various positions in the longitudinal direction of the cannula tube by means of a fastening device releasable at least for a limited axial displacement and in use the position of the cannula tube or the end of the cannula tube in the trachea of the patient is defined by the shield bearing against the neck of the patient, wherein the fastening device has at least one actuating element and can be brought into releasable clamping engagement with the outside of the cannula tube, **characterised in that** an arresting device (10) is provided for fixing the shield besides the fastening device (1) and the arresting device also has at least one actuating element (11) and can be brought into releasable engagement with the fastening device (1) and secures and/or reinforces the clamping engagement.

2. A tracheotomy cannula according to claim 1 **characterised in that** the at least one actuating element of the fastening device (1) and the at least one actuating element (11) of the arresting device (10) are independent of each other, wherein actuation of the actuating elements is effected by different mechanisms.

3. A tracheotomy cannula according to claim 1 or claim 2 **characterised in that** the arresting device (10) has a lever pivotable about an axis as the actuating element (11).

4. A tracheotomy cannula according to one of the preceding claims **characterised in that** the arresting device (10) has an actuating element (11) and the actuating element is movable beyond a dead centre point and/or after overcoming the dead centre point latches in a position in which the arresting device is in engagement with the fastening device (1) and/or with at least one actuating element of the fastening device.

5. A tracheotomy cannula according to one of the preceding claims **characterised in that** the arresting device (10) can be brought into releasable engagement with the fastening device (1) and/or with at least one actuating element of the fastening device and the fastening device and/or the at least one actuating element of the fastening device is blockable thereby.

6. A tracheotomy cannula according to one of the preceding claims **characterised in that** the fastening device (1) has a pushbutton (6) as the actuating element.

7. A tracheotomy cannula according to one of the preceding claims **characterised in that** the fastening device (1) is biased into clamping engagement with the outside of the cannula tube.

8. A tracheotomy cannula according to one of the preceding claims **characterised in that** the fastening device (1) comprises at least two ringshaped holding portions which at least partially embrace the cannula and which are displaceable relative to each other perpendicularly to their ring axes.

9. A tracheotomy cannula according to one of claims 1 to 7 **characterised in that** the fastening device (1) comprises at least two ringshaped holding portions which at least partially embrace the cannula, wherein the holding portions are pivotable about a third axis which is parallel to the two ring axes but does not coincide with one of said ring axes.

10. A tracheotomy cannula according to one of the preceding claims **characterised in that** at least a part of the fastening device (1), which part is in engagement with the cannula tube, and/or a part of the arresting device (10), is of a toothed configuration.

11. A tracheotomy cannula according to one of the preceding claims **characterised in that** the shield is fastened to the fastening device (1).

12. A tracheotomy cannula according to claim 11 **characterised in that** the shield has an opening, the diameter of which is larger than the diameter of the tracheotomy cannula and at least the fastening device (1) has a cylindrical projection, wherein the opening of the shield is substantially concentrically oriented relative to the cylindrical projection of the fastening device.

13. A tracheotomy cannula according to claim 12 **characterised in that** the cannula tube comprises an inner cannula and an outer cannula, wherein the inner cannula can be inserted through the shield and the cylindrical projection into the outer cannula.

14. A tracheotomy cannula according to one of claims 12 and 13 **characterised in that** at its free end the cylindrical projection has a radially outwardly protruding holding flange, for fastening the shield.

## Revendications

1. Canule de trachéotomie comprenant un tube de canule doté d'une collerette disposée sur le tube de canule, la collerette étant disposée sur la canule de manière à pouvoir être fixée dans la direction longitudinale du tube de canule dans différentes positions à l'aide d'un dispositif de fixation détachable au moins pour un déplacement axial limité et, lors de l'utilisation, la position du tube de canule ou de l'extrémité du tube de canule dans la trachée artère du patient étant définie par l'appui de la collerette contre le cou du patient, le dispositif de fixation comprenant au moins un élément d'actionnement et pouvant être amené en contact de serrage détachable avec le côté extérieur du tube de canule, **caractérisée en ce qu'**un dispositif d'arrêt (10) est prévu pour la fixation de la collerette en plus du dispositif de fixation (1) et le dispositif d'arrêt comprend également au moins un élément d'actionnement (11) et peut être amené en contact détachable avec le dispositif de fixation (1) et bloque et/ou renforce le contact de serrage.

2. Canule de trachéotomie selon la revendication 1, **caractérisée en ce que** le ou les éléments d'actionnement du dispositif de fixation (1) et le ou les éléments d'actionnement (1) du dispositif d'arrêt (10) sont indépendants les uns des autres, l'actionnement des éléments d'actionnement s'effectuant par différents mécanismes.

3. Canule de trachéotomie selon la revendication 1 ou la revendication 2, **caractérisée en ce que** le dispositif d'arrêt (10) comprend comme élément d'actionnement (11) un levier pivotant autour d'un axe.

4. Canule de trachéotomie selon l'une quelconque des revendications précédentes, **caractérisée en ce que** le dispositif d'arrêt (10) comprend un élément d'actionnement (11) et l'élément d'actionnement est mobile au-dessus d'un point mort et/ou après avoir surmonté le point mort s'enclenche dans une position dans laquelle le dispositif d'arrêt est en contact avec le dispositif de fixation (1) et/ou avec au moins un élément d'actionnement du dispositif de fixation.

5. Canule de trachéotomie selon l'une quelconque des revendications précédentes, **caractérisée en ce que** le dispositif d'arrêt (10) peut être amené en contact détachable avec le dispositif de fixation (1) et/ou avec au moins un élément d'actionnement du dispositif de fixation et le dispositif de fixation et/ou le ou les éléments d'actionnement du dispositif de fixation peuvent être bloqués de cette façon.

6. Canule de trachéotomie selon l'une quelconque des revendications précédentes, **caractérisée en ce que** le dispositif de fixation (1) comprend comme élément d'actionnement un bouton poussoir (6).

7. Canule de trachéotomie selon l'une quelconque des revendications précédentes, **caractérisée en ce que** le dispositif de fixation (1) est précontraint en contact de serrage avec le côté extérieur du tube de canule.

8. Canule de trachéotomie selon l'une quelconque des revendications précédentes, **caractérisée en ce que** le dispositif de fixation (1) est composé d'au moins deux parties de retenue annulaires entourant au moins en partie la canule et mobiles l'une par rapport à l'autre perpendiculairement à leurs axes annulaires.

9. Canule de trachéotomie selon l'une quelconque des revendications 1 à 7, **caractérisée en ce que** le dispositif de fixation (1) est composé d'au moins deux parties de retenue annulaires entourant au moins en partie la canule, les parties de retenue pouvant pivoter autour d'un troisième axe parallèle aux deux axes annulaires mais ne coïncidant pas avec un de ces axes annulaires.

10. Canule de trachéotomie selon l'une quelconque des revendications précédentes, **caractérisée en ce qu'**au moins une partie du dispositif de fixation (1) en contact avec le tube de canule et/ou une partie du dispositif d'arrêt (10) sont dentées.

11. Canule de trachéotomie selon l'une quelconque des revendications précédentes, **caractérisée en ce que** la collerette est fixée sur le dispositif de fixation (1).

12. Canule de trachéotomie selon la revendication 11, **caractérisée en ce que** la collerette comprend une ouverture dont le diamètre est supérieur au diamètre de la canule de trachéotomie et au moins les dispositifs de fixation (1) présentent un épaulement cylindrique, l'ouverture de la collerette étant orientée de manière sensiblement concentrique par rapport à l'épaulement cylindrique du dispositif de fixation.

13. Canule de trachéotomie selon la revendication 12, **caractérisée en ce que** le tube de canule est composé d'une canule intérieure et d'une canule extérieure, la canule intérieure pouvant être introduite dans la canule extérieure en passant par la collerette et l'épaulement cylindrique.

14. Canule de trachéotomie selon l'une quelconque des revendications 12 à 13, **caractérisée en ce que** l'épaulement cylindrique comprend sur son extrémité libre une bride de retenue faisant radialement saillie vers l'extérieur pour la fixation de la collerette.
